# EUROPEAN PATENT APPLICATION

(11) **EP 4 810 005 A1**
(43) Date of publication of application: **23.09.2026**
(21) Application number: 25165409.1
(22) Date of filing: 21.03.2025
(51) Int. Cl.: A61K 8/34, A61Q 17/04, A61K 8/35, A61K 8/37, A61K 8/41, A61K 8/49

(54) **SPF BOOSTER OF SOLID ORGANIC UV FILTER(S)**

(71) Applicant: Oleon N.V., 9940 Ertvelde (BE)
(72) Inventor: PUPPETT, Mauro, 60350 VIEUX MOULIN (FR)
(74) Representative: Santarelli

(57) **Abstract**

The present invention relates to an use of a specific compound as SPF booster, and to compositions comprising it with solid organic UV filter(s), and the use of such compositions as sunscreen products.

## Description

The present invention relates to an use of a specific compound as SPF booster, and to compositions comprising it with solubilized solid organic UV filter(s), processes for preparing emulsions, and the use of such compositions as UV protection products, also called sunscreen products.

SPF stands for Sun Protection Factor. It is usually indicated on sunscreen products and followed by a number indicating how well the sunscreen protects skin against sunburn.

As the SPF value increases, sunburn protection increases.

UV filters are compounds that protect against the harmful effects of the ultraviolet rays from the sun. They are commonly used in cosmetic and/or dermatological products in order to protect the skin from UV rays (protection against UV-A and/or UV-B rays).

There are two types of UV filters: organic filters and inorganic (or mineral) filters. Among the organic filters, some are in solid form (for example in the form of powder) and others are in liquid form at 25°C and under normal pressure conditions.

Each UV filter protects against specific UV-A and/or UV-B rays.

Also, to obtain a high protection against UV rays, a mixture of UV filters in large amount, is usually used in sunscreen products.

However, there is a wish to reduce the quantity of UV filters in cosmetic products.

Indeed, the UV filters may present some health and environmental concerns. In addition, they are the most costly part of a sunscreen product formulation.

Most of UV filters are organic, and most of them are solid. These solid organic UV filters are lipophilic, so they are soluble in oils and/or non-polar solvents.

To achieve the best SPF, while minimizing the quantity of UV filters, it is important to solubilize solid organic UV filters.

Some molecules defined as SPF boosters can also be used to increase SPF, without the need to increase UV filter content.

A SPF Booster is by itself generally inactive as UV-protectant, but in the presence of a UV filter(s) is capable of increasing the UV protective activity. It enables therefore a reduction of the UV filter(s) concentration while maintaining or boosting the SPF performance.

In particular, the addition of a SPF booster to solubilized solid organic UV filter(s), boosts SPF value of the solubilized solid organic UV filter(s).

A SPF booster appears therefore a good solution for reducing the quantity of UV filters in a formulation of a sunscreen product, to obtain safer and performing sunscreen products. This represents also an important economic advantage for formulators.

The main SPF boosters currently on the market are synthetic polymers and/or waxes such as Styrene/Acrylates Copolymer (and) Acrylates Copolymer, Polyethylene (and) Polycyclopentadiene (and) Stearoxy Dimethicone, and Bis-PEG-12 Dimethicone Beeswax.

WO20165071336A1 discloses a polyacrylate booster comprising at least one acrylates copolymer having a weight average molecular weight ranging from about 75,000 to 140,000 g/mol and a Tg ranging from -20 to 50.

WO2021001029A1 describes a mixture of beeswax and at least one lactylate ester.

More recently, EP4245291A1 describes a SPF booster based on sustainable raw materials, which is a mixture of a wax with glycerides based on C12-C20 fatty acids.

However, customers are looking to return to more natural compositions that respect the environment and their health.

That is why, there is still a need for new biobased SPF boosters, which enhance the UV protection of solid organic UV filters.

The Applicant surprisingly found that a specific emollient could efficiently improve the SPF of solid organic UV filter(s) solubilized in another emollient.

Accordingly, the present invention relates to an use of isocetyl isoarachidol as a SPF booster.

Isocetyl isoarachidol is a lipophilic alcohol.

Isocetyl isoarachidol is known as an emollient.

In the cosmetic field, it is known to use some emollients to solubilize solid organic UV filters.

Due to its significantly higher viscosity than the viscosities of emollients generally used as solubilizers, isocetyl isoarachidol is not used as a solubilizer of solid organic UV filters.

Preferably, isocetyl isoarachidol is used as SPF booster in a composition comprising solubilized solid organic UV filter(s). In particular, the solid organic UV filter(s) is/are solubilized in emollient(s) other than isocetyl isoarachidol.

The present invention also relates to an composition comprising isocetyl isoarachidol, emollient(s) other than isocetyl isoarachidol, and solid organic UV filter(s).

In the composition according to the invention, the solid organic UV filter(s) is/are preferably solubilized in emollient(s) other than isocetyl isoarachidol.

Preferably, the emollient(s) other than isocetyl isoarachidol, is/are chosen among the group consisting of C12-C15 alkyl benzoate, diisopropyl adipate, propylene glycol diheptanoate, isoamyl laurate, dibutyl adipate, butylene glycol esters, ethylhexyl salicylate, succinates, and mixtures thereof.

More preferably, the emollient(s) other than isocetyl isoarachidol is/are chosen among the group consisting of C12-C15 alkyl benzoate, diisopropyl adipate, propylene glycol diheptanoate, isoamyl laurate, dibutyl adipate, and mixtures thereof.

The total quantity of emollient(s) other than isocetyl isoarachidol is at least equal to the quantity required to completely solubilize the solid organic UV filter(s) present in the composition.

Preferably, the solid organic UV filter(s) is/are chosen among the group consisting of bis-ethylhexyloxyphenol methoxyphenyl triazine, butyl methoxydibenzoylmethane, diethylamino hydroxybenzoyl hexyl benzoate, diethylhexyl butamido triazone, ethylhexyl triazone, benzophenone-3, and mixtures thereof.

More preferably, the solid organic UV filter(s) is/are chosen among the group consisting of bis-ethylhexyloxyphenol methoxyphenyl triazine, butyl methoxydibenzoylmethane, diethylamino hydroxybenzoyl hexyl benzoate, diethylhexyl butamido triazone, ethylhexyl triazone, and mixtures thereof.

Preferably, the total quantity of solid organic UV filter(s) is of at least 5%, more preferably of at least 8%, even more preferably of at least 10% by weight based on the weight of the composition.

Preferably, the total quantity of solid organic UV filter(s) is of at most 40%, more preferably of at most 30%, even more preferably of at most 25% by weight based on the weight of the composition.

Preferably, the total quantity of solid organic UV filter(s) is comprised between 5 and 40% by weight, more preferably between 5 and 30%, such as between 10 and 30%, even more preferably between 10 and 25% by weight based on the weight of the composition.

By "total quantity of X", it is intended to mean the quantity of all X present in the composition.

In the present application, unless otherwise indicated, all ranges of values used are to be understood as being inclusive limits.

Preferably, at least two solid organic UV filters are present in the composition according to the invention.

Preferably, the quantity of isocetyl isoarachidol is of at least 1%, more preferably of at least 3%, even more preferably of at least 5% by weight based on the weight of the composition.

Preferably, the quantity of isocetyl isoarachidol is of at most 25%, more preferably of at most 20%, even more preferably of at most 15%, such as at most 10% by weight based on the weight of the composition.

Preferably, the quantity of isocetyl isoarachidol is comprised between 1 and 25%, such as between 3 and 25% and between 5 and 25%, more preferably between 3 and 20%, such as between 3 and 15% and between 3 and 10%, even more preferably between 5 and 15%, such as between 5 and 10% by weight based on the weight of the composition.

Preferably, the composition according to the invention is a homogeneous liquid. More particularly, the composition according to the invention is free of dispersion of lipophobic ingredient in powder form.

A preferred composition according to the invention, comprises:
- at least 1 wt% of isocetyl isoarachidol;
- at least 5 wt% of solid organic UV filter(s);
- at least 5 wt% of emollient(s) other than isocetyl isoarachidol; weight percentages being base on the weight of the composition.

In a first embodiment, the composition according to the invention is free of water.

Preferably, the total quantity of emollient(s) other than isocetyl isoarachidol is of at least 35%, more preferably of at least 40%, even more preferably of at least 50% by weight based on the weight of the composition.

Preferably, the total quantity of emollient(s) other than isocetyl isoarachidol is of at most 94%, more preferably of at most 92%, even more preferably of at most 90%, such as at most 85% by weight based on the weight of the composition.

Preferably, the total quantity of emollient(s) other than isocetyl isoarachidol is comprised between 35 and 94%, such as between 35 and 92%, more preferably between 40 and 92%, such as between 50 and 92%, even more preferably between 50 and 90%, such as between 55 and 90% by weight based on the weight of the composition.

A preferred composition according to the first embodiment of the composition according to the invention, comprises:
- 1-25 wt% of isocetyl isoarachidol;
- 5-40 wt% of solid organic UV filter(s);
- 35-94 wt% of emollient(s) other than isocetyl isoarachidol;
weight percentages being base on the weight of the composition.

A particularly preferred composition according to the first embodiment of the composition according to the invention, comprises:
- 3-15 wt% of isocetyl isoarachidol;
- 5-35 wt% of solid organic UV filter(s);
- 50-92 wt% of emollient(s) other than isocetyl isoarachidol;
weight percentages being base on the weight of the composition.

In a second embodiment, the composition according to the invention further comprises water.

Preferably, the composition according to the second embodiment comprises at least 40% by weight of water based on the weight of the composition.

Preferably, the composition according to the second embodiment comprises at most 75% by weight of water based on the weight of the composition.

Preferably, the composition according to the second embodiment comprises between 40 and 75% by weight of water based on the weight of the composition.

Preferably, the total quantity of emollient(s) other than isocetyl isoarachidol is of at least 5%, more preferably of at least 8%, even more preferably of at least 10% by weight based on the weight of the composition.

Preferably, the total quantity of emollient(s) other than isocetyl isoarachidol is of at most 40%, more preferably of at most 35%, even more preferably of at most 30% by weight based on the weight of the composition.

Preferably, the total quantity of emollient(s) other than isocetyl isoarachidol is comprised between 5 and 40%, more preferably between 5 and 35%, such as between 8 and 35%, even more preferably between 10 and 30% by weight based on the weight of the composition.

Advantageously, the composition according to the second embodiment, further comprises emulsifier(s).

The emulsifier(s) can be any emulsifier known by the skilled person.

Preferably, the composition according to the second embodiment is in the form of an emulsion. The emulsion can be an oil-in-water emulsion or a water-in-oil emulsion.

Preferably, for composition in form of oil-in-water emulsion, the emulsifier(s) present an HLB value comprised between 8 and 18.

More particularly, for an oil-in-water emulsion, the emulsifier(s) having a HLB value comprised between 8 and 18 is/are preferably chosen among the group consisting of glyceryl stearate and PEG-100 stearate; fatty alcohol ethoxylates; fatty acid ethoxylates; sorbitan esters; glyceryl esters; PEG-esters; anionic emulsifiers, such as sulfates, sulfonates, and phosphates; natural waxes; synthetic waxes; and mixtures thereof.

Preferably, for composition in form of water-in-oil emulsion, the emulsifier(s) present an HLB value comprised between 3 and 6.

More particularly, for a water-in-oil emulsion, the emulsifier(s) having a HLB value between 3 and 6 is/are preferably chosen among the group consisting of non-ionic surfactants, such as polyglycerol esters, sorbitan oleate, glyceryl stearate, and PEG esters; silicone-based emulsifiers, such as PEG/PPG-10/1 dimethicone, and cetyl dimethicone copolyol; fatty alcohols, such as stearyl alcohol, and cetyl alcohol; and mixtures thereof.

By "HLB" (Hydrophilic-Lipophilic Balance), it is intended to mean the balance between the size and the strength of the hydrophilic group and the size and strength of the lipophile group of the surfactant. The HLB according to GRIFFIN is defined in J. Soc. Cosm. Chem. 1954 (volume 5), pages 249-256.

Preferably, the total quantity of emulsifier(s) is of at least 1% by weight, more preferably of at least 2%, even more preferably of at least 3% by weight based on the weight of the composition.

Preferably, the total quantity of emulsifier(s) is of at most 10%, more preferably of at most 8%, even more preferably of at most 6% by weight based on the weight of the composition.

Preferably, the total quantity of emulsifier(s) is comprised between 1 and 10%, more preferably between 2 and 8%, even more preferably between 3 and 8%, such as between 3 and 6% by weight based on the weight of the composition.

A preferred composition according to the second embodiment of the composition according to the invention, comprises:
- at least 40 wt% of water;
- 1-25 wt% of isocetyl isoarachidol;
- 5-30 wt% of solid organic UV filter(s);
- 5-35 wt% of emollient(s) other than isocetyl isoarachidol;
- 1-8 wt% of emulsifier(s);
weight percentages being base on the weight of the composition.

A particularly preferred composition according to the second embodiment of the composition according to the invention, comprises:
- at least 50% by weight of water;
- 3-20% by weight of isocetyl isoarachidol;
- 5-25% by weight of solid organic UV filter(s);
- 10-30% by weight of emollient(s) other than isocetyl isoarachidol;
- 1-8% by weight of emulsifier(s);
weight percentages being based on the weight of the composition.

The composition in the form of an emulsion according to the second embodiment of the composition according to the invention, may further comprises a solvent other than water.

More particularly, the solvent other than water is chosen among the group consisting of ethanol, glycerin, and mixtures thereof.

Advantageously, the composition according to the invention, further comprises thickening agent(s) and/or preservative(s).

Preferably, the total quantity of the thickening agent(s) is/are of at least 0.1% by weight based on the weight of the composition.

Preferably, the total quantity of the thickening agent(s) is/are of at most 1%, more preferably of at most 0.5%, even more preferably of at most 0.4% such as of at most 0.3% by weight based on the weight of the composition.

Preferably, the total quantity of the thickening agent(s) is/are comprised between 0.1 and 1%, more preferably between 0.1 and 0.5%, even more preferably between 0.1 and 0.4%, such as between 0.1 and 0.3% by weight based on the weight of the composition.

The thickening agent(s) is/are preferably chosen among the group consisting of polymers of acrylic acid such as carbomer; gums such as xanthan and carrageenan; polysaccharides such as cellulose and starch; cocamide DEA; and mixtures thereof. In particular, the thickening agent(s) is a polymer of acrylic acid such as carbomer.

The preservative(s) is/are preferably chosen among the group consisting of phenoxyethanol, glycol ethers, benzoic acid, sorbic acid, salicylic acid, glycols, parabens, thiazolinones, ethylhexylglycerin, aldehydes, and mixtures thereof.

Preferably, the total quantity of preservative(s) is/are of at least 0.5% by weight based on the weight of the composition.

The present invention also concerns an use of the composition according to the invention, as a cosmetic product.

The cosmetic product can be in the form of a gel, a gel cream, a spray or a lotion.

The cosmetic product can be any product comprising one or more solid organic UV filter(s), in particular a sunscreen product.

The cosmetic product can be a skin product, a hair product, or a product for make-up such as a lipstick.

The cosmetic product can further comprise additional ingredients that are typically used in cosmetics, such as an active ingredient and/or a perfume.

More particularly, the active ingredient is chosen among the group consisting of allantoin, tocopherol, ascorbic acid, ascorbyl palmitate, vitamin D, polyphenols, flavonoids, and mixtures thereof.

The present invention also concerns a process for preparing a composition in the form of an oil-in-water emulsion according to the second embodiment of the composition according to the invention, comprising a step of emulsification:
- of an oil phase comprising isocetyl isoarachidol, emollient(s) other than isocetyl isoarachidol, and solid organic UV filter(s);
- in an aqueous phase.

The present invention also concerns a process for preparing a composition in the form of a water-in-oil emulsion according to the second embodiment of the composition according to the invention, comprising a step of emulsification:
- of an aqueous phase;
- in an oil phase comprising isocetyl isoarachidol, emollient(s) other than isocetyl isoarachidol, and solid organic UV filter(s).

In the processes according to the invention, the isocetyl isoarachidol, the solid organic UV filter(s), and the emollient(s) other than isocetyl isoarachidol, are as described above, including the preferential and advantageous features.

Before the step of emulsification, the oil phase and/or the aqueous phase is/are preferably heated at a temperature of at most 90°C, more preferably at a temperature of at most 80°C.

The oil phase may further comprise emulsifier(s) and/or active ingredient(s).

Besides water, the aqueous phase may further comprise emulsifier(s), a solvent other than water such as described above, and/or active ingredient(s).

As shown in Example 2, the oil-in-water emulsions according to the invention may present a high SPF by improving by at least 15% and up to 68% the SPF value, due to the presence of isocetyl isoarachidol in the emulsions, which acts as a SPF booster.

The present invention also relates to a method for boosting the SPF value of a composition comprising solid organic UV filter(s) and emollient(s) other than isocetyl isoarachidol, by adding isocetyl isoarachidol in said composition.

The isocetyl isoarachidol, the solid organic UV filter(s), the emollient(s) other than isocetyl isoarachidol, and the composition are as described above, including the preferential and advantageous features.

Preferably, at least 1%, more preferably at least 3%, even more preferably at least 5% by weight based on the weight of the composition, of isocetyl isoarachidol are added to the composition.

The SPF value of the composition is improved by at least 15%.

The invention is further described in the following Examples. It will be appreciated that the invention as claimed is not intended to be limited in any way by these examples.

### Chemicals used in Examples

- SPF booster:
   - isocetyl isoarachidol: Radiastar 1436 from Oleon;
- Emollients:
   - C12-1C5 alkyl benzoate: Finsolv TN from Innospec;
   - diisopropyl adipate: Schercemol DIA by Lubrizol;
   - propylene glycol diheptanoate: Jolee 7202 from Oleon;
- Solid organic UV filters:
   - bis-Ethylhexyloxyphenol metoxyphenyl triazine: Tinosorb S by BASF;
   - butyl methoxydibenzoylmethane (avobenzone) : Eusolex 9020 from Merck;
   - diethylamino hydroxybenzoyl hexyl benzoate : Uvinul A Plus from BASF;
   - diethylhexyl butamido triazone: Uvasorb HEB by 3V Sigma;
   - ethylhexyl triazone: Uvinul T150 from BASF;
- Emulsifier:
   - glyceryl stearate and PEG-100 stearate: Radia 7490 from Oleon;
- Thickening agent:
   - carbomer: Carbopol Ultrex 10 from Lubrizol;
- Preservatives:
   - phenoxyethanol from Arkema;
   - ethylhexyl glycerine: Lexgard E by Inolex.

### Example 1: Preparation of compositions in the form of oil-in-water emulsions

### 1.1 Sunscreen products E11 and E12 according to the invention and comparative sunscreen product CE1

Chemicals and their quantities are described in Table 1.

To prepare the oil-in-water emulsions E11, E12 and CE1, phase A was prepared by introducing into a main beaker the demineralized water, and under stirring the thickening agent.

Phase A was neutralized with a 20% solution of sodium hydroxide in demineralized water to obtain a pH comprised between 6 and 7.

Phase A was then heated to 80°C.

Phase B was prepared by introducing in a separate beaker, the emollient, the solid UV filters, the SPF booster, and the emulsifier, under stirring (800 rpm).

Phase B was then heated to 80°C.

Phase B was added to phase A and homogenized using an Ultra-Turrax at 9000 rpm.

The preservatives of phase C were then added to the homogenized mixture obtained.

The pH was controlled and adjusted if needed to 6-7 by adding a 20% solution of sodium hydroxide in demineralized water.

**Table 1: Formulation of oil-in-water emulsions E11 and E12 according to the invention and of comparative oil-in-water emulsion CE1**

| | | Function | CE1 (%w/w) | E11 (%w/w) | E12 (%w/w) |
|---|---|---|---|---|---|
| A | Demineralized water | solvent | 58.8 | 53.8 | 58.8 |
| | Carbomer | thickening agent | 0.1 | 0.1 | 0.1 |
| | NaOH, sol 20 wt% | pH modifier | appropriate amount | appropriate amount | appropriate amount |
| | C12-C15 alkyl benzoate | emollient | 20 | 20 | 15 |
| B | Isocetyl Isoarachidol | SPF booster | 0 | 5 | 5 |
| | Glyceryl stearate and PEG-100 stearate | emulsifier | 3 | 3 | 3 |
| | Bis-ethylhexyl-oxyphenol metoxyphenyl triazine | solid organic UV filter | 5 | 5 | 5 |
| | Butylmethoxydibenzoyl methane | solid organic UV filter | 3 | 3 | 3 |
| | Diethylamino hydroxybenzoyl hexyl benzoate | solid organic UV filter | 5 | 5 | 5 |
| | Diethylhexyl butamido triazone | solid organic UV filter | 1 | 1 | 1 |
| | Ethylhexyl triazone | solid organic UV filter | 3 | 3 | 3 |
| C | Phenoxyethanol | preservative | 0.6 | 0.6 | 0.6 |
| | Ethylhexyl glycerine | preservative | 0.5 | 0.5 | 0.5 |

### 1.2 Sunscreen products E21 and E22 according to the invention and comparative sunscreen product CE2

The oil-in-water emulsions E21, E22, and CE2 were prepared according to the method described in Example 1.1 using chemicals described in Table 2.

**Table 2: Formulation of oil-in-water emulsions E21 and E22 according to the invention and of comparative oil-in-water emulsion CE2**

| | | Function | CE2 (%w/w) | E21 (%w/w) | E22 (%w/w) |
|---|---|---|---|---|---|
| A | Demineralized water | solvent | 58.8 | 53.8 | 58.8 |
| | Carbomer | thickening agent | 0.1 | 0.1 | 0.1 |
| | NaOH, sol 20 wt% | pH modifier | appropriate amount | appropriate amount | appropriate amount |
| B | Diisopropyl adipate | emollient | 20 | 20 | 15 |
| | Isocetyl Isoarachidol | SPF booster | 0 | 5 | 5 |
| | Glyceryl stearate and PEG-100 stearate | emulsifier | 3 | 3 | 3 |
| | Bis-ethylhexyl-oxyphenol metoxyphenyl triazine | solid organic UV filter | 5 | 5 | 5 |
| | Butylmethoxydibenzoyl methane | solid organic UV filter | 3 | 3 | 3 |
| | Diethylamino hydroxybenzoyl hexyl benzoate | solid organic UV filter | 5 | 5 | 5 |
| | Diethylhexyl butamido triazone | solid organic UV filter | 1 | 1 | 1 |
| | Ethylhexyl triazone | Solid organic UV filter | 3 | 3 | 3 |
| C | Phenoxyethanol | preservative | 0.6 | 0.6 | 0.6 |
| | Ethylhexyl glycerine | preservative | 0.5 | 0.5 | 0.5 |

### 1.3 Sunscreen products E31 and E32 according to the invention and comparative sunscreen product CE3

The oil-in-water emulsions E3, CE31 and CE32 were prepared according to the method described in Example 1.1 using chemicals described in Table 3.

**Table 3: Formulation of oil-in-water emulsions E31 and E32 according to the invention and of comparative oil-in-water emulsion CE3**

| | | Function | CE3 (%w/w) | E31 (%w/w) | E32 (%w/w) |
|---|---|---|---|---|---|
| A | Demineralized water | solvent | 58.8 | 53.8 | 58.8 |
| | Carbomer | thickening agent | 0.1 | 0.1 | 0.1 |
| | NaOH, sol 20 wt% | pH modifier | appropriate amount | appropriate amount | appropriate amount |
| B | Propylene glycol diheptanoate | emollient | 20 | 20 | 15 |
| | Isocetyl Isoarachidol | SPF booster | 0 | 5 | 5 |
| | Glyceryl stearate and PEG-100 stearate | emulsifier | 3 | 3 | 3 |
| | Bis-ethylhexyl-oxyphenol metoxyphenyl triazine | solid organic UV filter | 5 | 5 | 5 |
| | Butylmethoxydibenzoyl methane | solid organic UV filter | 3 | 3 | 3 |
| | Diethylamino hydroxybenzoyl hexyl benzoate | solid organic UV filter | 5 | 5 | 5 |
| | Diethylhexyl butamido triazone | solid organic UV filter | 1 | 1 | 1 |
| | Ethylhexyl triazone | solid organic UV filter | 3 | 3 | 3 |
| C | Phenoxyethanol | preservative | 0.6 | 0.6 | 0.6 |
| | Ethylhexyl glycerine | preservative | 0.5 | 0.5 | 0.5 |

### Example 2: SPF boosting property of isocetyl isoarachidol

The SPF value of oil-in-water emulsions prepared in Example 1 were assed in-vitro, using a Labsphere 2000S with 5µm sand blasted plates.

Results are gathered in Table 4.

**Table 4: SPF values of oil-in-water emulsions E11-E12, E21-E22 and E31-E32 according to the invention and of comparative oil-in-water emulsions CE1-CE3**

| Oil-in-water emulsions | SPF value |
|---|---|
| CE1 | 54.1 |
| E11 | 68.26 |
| E12 | 64.84 |
| CE2 | 32.33 |
| E21 | 54.32 |
| E22 | 53.76 |
| CE3 | 42.9 |
| E31 | 68.23 |
| E32 | 61.22 |

It can be observed that adding 5 wt% of isocetyl isoarachidol or replacing 5 wt% of emollient with 5 wt% of isocetyl isoarachidol, increases the SPF values.

In emulsions comprising C12-C15 alkyl benzoate as emollient (CE1), the addition of 5 wt% of isocetyl isoarachidol (E11) or the replacement of 5 wt% of C12-C15 alkyl benzoate by isocetyl isoarachidol (E12), increases the SPF by 26.1% and 19.8% respectively.

In emulsions comprising diisopropyl adipate as emollient (CE2), the addition of 5 wt% of isocetyl isoarachidol (E21) or the replacement of 5 wt% of diisopropyl adipate by isocetyl isoarachidol (E22), increases the SPF by 68% and 66.2% respectively.

In emulsions comprising propylene glycol diheptanoate as emollient (CE3), the addition of 5 wt% of isocetyl isoarachidol (E31) or the replacement of 5 wt% of propylene glycol diheptanoate by isocetyl isoarachidol (E32), increases the SPF by 59% and 42.7% respectively.

It can be concluded that isocetyl isoarachidol exhibits a SPF boosting property.

## Claims

1. Use of isocetyl isoarachidol as a SPF booster.

2. Composition comprising isocetyl isoarachidol, emollient(s) other than isocetyl isoarachidol, and solid organic UV filter(s).

3. Composition according to claim 2, further comprising water.

4. Composition according to claim 3, further comprising emulsifier(s).

5. Composition according to any of claims 2 to 4, further comprising thickening agent(s) and/or preservative(s).

6. Use of the composition according to any of claims 2 to 5, as a cosmetic product.

7. Process for preparing a composition in the form of an oil-in-water emulsion according to any of claims 3 to 5, comprising a step of emulsification:
- of an oil phase comprising isocetyl isoarachidol, emollient(s) other than isocetyl isoarachidol, and solid organic UV filter(s);
- in an aqueous phase.

8. Process for preparing a composition in the form of a water-in-oil emulsion according to any of claims 3 to 5, comprising a step of emulsification:
- of an aqueous phase;
- in an oil phase comprising isocetyl isoarachidol, emollient(s) other than isocetyl isoarachidol, and solid organic UV filter(s).

9. Method for boosting the SPF value of a composition comprising solid organic UV filter(s) and emollient(s) other than isocetyl isoarachidol, by adding isocetyl isoarachidol in said composition.
